# EUROPEAN PATENT APPLICATION

(11) **EP 1 790 329 A1**
(43) Date of publication of application: **30.05.2007**
(21) Application number: 05772705.9
(22) Date of filing: 17.08.2005
(51) Int. Cl.: A61K 8/60, A61Q 19/00, A61K 31/704, A61Q 5/02, A61Q 19/10, A61P 17/16

(54) **HUMECTANT CONTAINING ALPHA-GLYCOSYL GLYCYRRHIZIN AS ACTIVE INGREDIANT AND USE THEREOF**

(30) Priority: 23.08.2004 JP 2004242769
(71) Applicant: KABUSHIKI KAISHA HAYASHIBARA SEIBUTSU KAGAKU KENKYUJO, Okayama-shi, Okayama 700-0907 (JP)
(72) Inventor: TATSUKAWA, Hiromi, c/o K.K. HAYASHIBARA SEIBUTSU, Okayama-si, Okayama 700-0907 (JP); MIYAKE, Toshio, c/o K.K. HAYASHIBARA SEIBUTSU, Okayama-shi, Okayama 700-0907 (JP)
(74) Representative: Daniels, Jeffrey Nicholas
(86) International application number: PCT/JP2005/014993
(87) International publication number: WO 2006/022173

(57) **Abstract**

The present invention has objects to provide a humectant applicable to preparations for external dermal agents, which has a moisturizing activity without causing unfavorable sticky feeling, and to provide an external dermal agent incorporated with the humectant. These objects are solved by providing a humectant containing α-glycosyl glycyrrhizin as an effective ingredient, which has a satisfactory feeling of use and preferable makeup finish, imparts a desired softness and tension to the skin, and has an improved humectancy without causing unfavorable sticky feeling; and by providing an external dermal agent incorporated with the humectant.

## Description

### Technical Field

The present invention relates to a humectant containing glycosyl glycyrrhizin composed of glycyrrhizin to which at least an equimolar glucose residue is bound via the α-linkage (called "α-glycosyl glycyrrhizin", hereinafter), and to an external dermal agent which imparts a desired moisturizing effect to the skin and hair and also imparts an improved skin-whitening effect and retrogradation-preventing effect to the skin by using the humectant.

### Background Art

Conditions requisite for beautiful skin are, for example, a desired skin moisture-level, softness, tension, and smoothness free of wrinkle, dullness, and fleck. These properties are recognized to be correlated with the skin, particularly, the moisture level in the cutidure and the activity of chrotoplast. It is also said that the moisture level in the capillus is one of the factors that influence on the quality of capillus. Thus, it has been said that imparting an adequate level of moisture to the skin and capillus is an important function of makeup cosmetics and basic cosmetics for beautiful skin, hair growth, etc., and there have been explored a variety of external dermal agents directed to maintain the moisture in the skin and to whiten the skin.

As such external dermal agents, a variety of humectants and moisture absorbents alone or in an appropriate combination thereof have been proposed, and there have been used, as such moisture absorbents, humectant components such as polyalcohols including glycerin and 1,3-butylene glycol, as well as hyaluronic acid and chitin; NMF-acting substances as natural humectant components derived from the skin; and proteins such as collagen and elastin. The fact that, as the progress in the recent dermatological science, ceramides as intercellular lipid substances have been revealed to have an important function of retaining the moisture level in the corneum has made use as humectants synthetic or extracted ceramides derived from natural sources. Various cosmetics incorporated with ingredients having a skin-whitening activity, such as lactic acid, arbutin, and ascorbic acid derivatives, are commercially available. However, the external dermal agents incorporated with these humectants or moisturizing agents have the following problems;
(1) Intercellular lipids such as ceramides used as humectants are costly and poor in solubility in solvents such as water, and this may result in difficulty of incorporating them into external dermal agents in an amount up to an effective concentration level;
(2) Moisture absorbents impart a sticky feeling to users and give no freshness;
(3) In the case of incorporating moisture absorbents or intercellular lipid humectants each alone, it could not exert a sufficient level of moisture-retaining effect, has a poor feeling in use and a lesser makeup retainability, and has difficulty of preparing external dermal agents which impart an ideal moisture absorbing and moisturizing activities to the skin. Further, in the case of using them in combination, their incompatibility and coloration restrict the form or the concentration of cosmetics;
(4) To attain a desired durability of cosmetic effect, hydrophobicity-imparted powders are generally used in makeup cosmetics prepared with a relatively large amount of powders, and such powders absorb sebum but have a poor moisture retainability, and this may tend to easily cause skin dryness; and
(5) Incorporation of substances with skin-whitening activity may induce stickiness or the like and lower the feeling of use or may induce white turbidity and sedimentation.

Therefore, further development of humectants for external dermal agents applicable to various forms of agents such as cosmetics for skin care, makeup, body care, and hair, which have a moisture-imparting activity and/or skin-whitening activity but have no stickiness.

While licorice extracts, glycyrrhizin contained therein and salts thereof, as well as derivatives prepared therewith are generally used in cosmetics as substances having an antiphlogistic activity (of. Japanese Patent Kokai No. 1281136/94) and in the field of food products as food additives, sweeteners, taste improvers, etc. (cf. *"The List of Notice of Statutes on The List* of Existing Food Additives Under Revisions to The Food Product Health Laws", edited by the Food Chemistry Division of Environmental Health Bureau of the Ministry of Health and Welfare, published by Japan Food Additives Association, pages 18, 19 and 30, 1996). Japanese Patent Kokai No. 870/83 discloses that saccharide-transferred glycyrrhizin and extracts from licorice containing the same can be used as a sweetener or flavoring substance in food products; cosmetics for oral cavity and lip such as lip creams, dentifrices, and rouges; pharmaceuticals; and favorites foods including cigarettes and tobaccos, and they can be used in pharmaceuticals directed to anti-inflammatory, controlling of intestinal disorder, expectorating, antitussive, etc. These literatures, however, neither disclose nor suggest the fact that α-glycosyl glycyrrhizin is suitably used as a humectant and/or skin-whitening agent in external dermal agents.

### Disclosure of Invention

Under the above circumstances, the present invention has objects to provide a humectant which has applicability to various forms of external dermal agents, as well as having improved humectancy, no stickiness, satisfactory usability, improved skin-whitening effect, and satisfactory retrogradation-preventing effect on the skin; and to provide an external dermal agent incorporated with the humectant.

As a result of energetic researches, the present inventors found that α-glycosyl glycyrrhizin has an improved moisturizing activity and exerts a satisfactory moisturizing effect while imparting an improved feeling of use without stickiness when used in various forms of external dermal agents. They also found that humectants containing α-glycosyl glycyrrhizin have advantageous skin-whitening effect, cell activating effect, and retrogradation-preventing effect on the skin. Accordingly, they accomplished the above objects of the present invention by providing a humectant containing α-glycosyl glycyrrhizin as an effective ingredient and an external dermal agent incorporated with the humectant.

### Best Mode for Carrying Out the Invention

The term "α-glycosyl glycyrrhizin" as referred to as in the present invention means a compound, composed of glycyrrhizin to which at least an equimolar glucose residue is bound via the α-linkage, and/or salts thereof; and includes those which contain α-glyoosyl glycyrrhizin and unglycosylated glycyrrhizin and/or salts thereof. Concrete examples of such are α-oligosyl glycyrrhizin including α-glucosyl glycyrrhizin, α-maltosyl glycyrrhizin, and α-maltotriosyl glycyrrhizin, which are respectively composed of glycyrrhizin to which one mole, two moles, three moles, or more glucose residues are bound.

The α-glycosyl glycyrrhizin used in the present invention should not specifically be restricted to those of specific origins and preparation methods and it includes any of those which are prepared by fermentation, enzymatic, organic synthetic methods, etc. Examples of such are extracts containing glycyrrhizin obtained by extracting roots and/or rhizomes of licorice, i.e., papilionaceous perennial plants, such as *Glycyrrhiza uralensis Fisher, Glycyrrhiza inflata BATALIN, and Glycyrrhiza glabra LINNE;* purified ones prepared by subjecting mixture solutions of purified products of the above extracts and amylaceous substances such as dextrins to the action of α-glycosyl saccharide-transferring enzymes such as cyclomaltodextrin glucanotransferase, etc., to glycosylate glycyrrhizin, and purifying the formed glycosyl glycyrrhizin using macroporous synthetic adsorption resins such as "DIAION HP-10" and "DIAION HP-20", commercialized by Mitsubishi Chemical Corporation, Tokyo, Japan; and "AMBERLITE XAD-2", "AMBERLITE XAD-7", etc., commercialized by Rohm & Hass Company, Philadelphia, PA., USA; or others with an improved purity of α-glycosyl glycyrrhizin, prepared by removing the coexisting glycyrrhizin. Among which, particularly desired are the ones rich in α-glucosyl glycyrrhizin composed of glycyrrhizin to which an equimolar glucose residue is bound, obtained by subjecting the above-identified α-glycosyl glycyrrhizin to the action of glucoamylase, etc., or to purification using resins, have a relatively high moisturizing effect and a relatively strong biological activity such as skin whitening activity and retrogradation preventing activity. As the humectant containing α-glycosyl glycyrrhizin as an effective ingredient according to the present invention, those which contain glycyrrhizin and α-glycosyl glycyrrhizin in a total amount of at least 80% by weight (throughout the specification "% by weight" is abbreviated as "%", unless specified otherwise), preferably, at least 90%, and more preferably, at least 95%, can be advantageously used. Considering moisturizing activity, the α-glycosyl glycyrrhizin advantageously used in the present invention includes those which at least 50%, preferably, at least 60%, and more preferably, at least 80% of the total amount of glycyrrhizin is glycosylated. The α-glycosyl glycyrrhizin advantageously used in the present invention are those which at least 60%, preferably, at least 80%, and more preferably, at least 90% of α-glycosyl glycyrrhizin is α-glucosyl glycyrrhizin, in view of physiological activities such as skin-whitening activity and retrogradation-preventing activity.

The above-mentioned α-glycosyl glycyrrhizin preparations can be used intact as the humectant of the present invention or used in the form of a preparation produced by mixing with one or more materials selected from the later described substances that can be incorporated into external dermal agents, depending on use.

Since the α-glycosyl glycyrrhizin according to the present invention inhibits both the damage of cutaneous cells from ultraviolet rays, etc., and successively induced inflammatory, it can be advantageously used in external dermal agents as an anti-inflammatory, skin preventive, retrogradation-preventing agent for the skin, improver for crease or fine crease, and agent for inhibiting the generation of such skin disorders.

The α-glycosyl saccharide-transferring enzyme used in preparing the α-glycosyl glycyrrhizin according to the present invention should not specifically be restricted as long as it glycosylates glycyrrhizin. Concrete examples of such include cyclomaltodextrin glucanotransferase as disclosed in Japanese Patent Kokai No. 870/83. Among which, cyclomaltodextrin glucanotransferase derived from *Bacillus stearothermophilus* can be advantageously used because of its relatively high thermostability and transferring efficiency of glucose to glycyrrhizin.

The humectant containing α-glycosyl glycyrrhizin as an effective ingredient of the present invention can be applicable for various forms of agents such as basic cosmetics or ones for skin care; cosmetics for makeup, body care, hair, and mouth care; soaps; and bath salts. In addition to the above-mentioned cosmetics, the external dermal agents as referred to as in the present invention include quasi-drugs, pharmaceuticals, and daily necessities and miscellaneous goods such as detergents, which are used in such a manner of being contacted with the skin or the mucosa. The content of the humectant of the present invention to be incorporated into external dermal agents is usually selected from the range of 0.001 to 20% to the total amount of each agent; a comfortable moisturizing feeling can be attained when added in an amount of at least 0.001%, and the effect becomes clear with the addition of at least 0.01% and it becomes remarkable with the addition of 0.5 to 10%. The upper limit of the humectant to be added should not specifically be restricted as long as it does not affect the property and the function of the desired external dermal agents. Considering physiological functions such as skin-whitening effect, cell-activating effect, and retrogradation-preventing effect on the skin, the humectant should preferably be added in an amount of at least 0.001%, and the effect becomes clear with the addition of at least 0.01% and it becomes more remarkable with the addition of at least 0.1%.

The external dermal agents, to which the humectant of the present invention is applicable, include any of conventional forms of products, for example, cleansing cosmetics such as a cosmetic soap, face wash, shampoo, and rinse; basic cosmetics such as a cream, lotion, toilet water, cosmetic oil, and pack; makeup cosmetics such as a foundation, rouge, face powder/liquid/paste/oil, eye shadow, eye liner, mascara, cheek rouge, and eye makeup cosmetic; suntan/sunburn cosmetics; mouth cosmetics such as a dentifrice; and detergents. The cosmetics according to the present invention can be formed into various forms of products such as those in the form of a mass, pencil, stick, milky lotion, cream, liquid, powder, gel, mousse, or spray.

The humectant of the present invention can be incorporated into the desired external dermal agents at any steps of their processings from their material stage to their final product stage. Concrete examples of methods for such purpose are conventional techniques of mixing, kneading, dissolving, melting, spreading, suspending, emulsifying, treating for reversed micelle, permeating, crystallizing, sprinkling, applying, spraying, injecting, soaking, solidifying, and supporting, one or more of which can be used in an appropriate combination.

Since the humectant of the present invention has a satisfactory moisturizing activity and adhering activity and retains the skin in a moistened and fresh condition without causing stickiness, it can prepare cosmetics for finishing with an improved feeling of use when used in eye shadows, mascaras, foundations, etc. Thus, the humectant can be advantageously used as a humectant in these cosmetics. The humectant of the present invention also has a skin-whitening, cell activating, and anti-inflammatory activities, and it can be used to prepare external dermal agents that inhibit pigmentation in the skin or the lip damaged by ultraviolet rays or other factors or being in an inflammatory condition, improve the cellular metabolism of the skin, inhibit the inflammation in the skin, promote the recovery of damaged or inflammatory skin to the normal skin, and prevent the retrogradation of the skin.

Since the humectant of the present invention has a satisfactory compatibility with substances used in cosmetics, for example, emulsifiers, and solvents and low molecular polyols including ethanol and 1,2-pentanediol; and high molecules such as polyethylene glycol and carboxyvinylpolymer, it can be incorporated into external dermal agents in various forms.

The humectant of the present invention can be also incorporated into external dermal agents in combination with one or more substances selected from those which have a blood circulation improving activity, anti-inflammatory activity, antiseptic activity, moisture-retaining activity, skin-whitening activity, ultraviolet rays absorbing activity, ultraviolet rays scattering activity, astringent activity, anti-crease activity, antioxidant activity, cell-activating activity, anti-retrogradation activity, hair-growth activity, hair-regeneration activity, and percutaneous absorption promoting activity. Depending on purpose, the humectant of the present invention can be incorporated into external dermal agents in combination with any one or more conventional substances arbitrarily added to external dermal agents other than those with the above-identified activities. Examples of substances usable in external dermal agents in combination with the humectant of the present invention are propylene glycol, dipropylene glycol, 1,3-butylene glycol, glycerin, maltitol, sorbitol, α,α-trehalose, α,α-trehalose, α,α-trehalose, saccharide derivatives of α,α-trehalose including α-glucosyl α,α-trehalose, α-maltosyl α,α-trehalose, α-maltotriosyl α,α-trehalose and the like, saccharides containing the above derivatives, cyclic tetrasaccharide as disclosed in International Patent Publication No. WO 02/10361, cyclodextrins, isomalto-oligosaccharides, lactosucrose, sodium hyaluronate, pyrrolidone-carboxylates; svlid-/semi-solid oily components such as ceramide, petrolatum, lanoline, ceresin, silicone wax, microcrystalline wax, carnauba wax, candelillan wax, higher fatty acids, and higher alcohols; liquid oily components such as squalane, liquid petrolatum, dimethylpolysiloxane, methylphenylpolysiloxane, ester oils, and triglycerides; vitamins and derivatives thereof such as vitamin E, vitamin E acetate, L-ascorbic acid, phosphate and sulfate of L-ascorbic acid, L-ascorbic acid 2-glucoside, rutin, glycosyl rutin, hesperidin, glycosyl hesperidin, naringin, glycosyl naringin, esculetin, esculin, and glycosyl esculin; components from plants and animals such as herbs and royal jelly including coenzyme Q 10, AMP, ADP, ATP, isoflavones, glycosyl isoflavone, propolis, indigos, and Chinese parsley, as well as extracts thereof; antibiotics, physiologically active substances, pharmaceuticals, antioxidants, preservatives, flavors; pH-controlling agents such as citric acid, sodium citrate, lactic acid, sodium lactate, potassium lactate, sodium secondary phosphate, sodium hydroxide, and potassium hydroxide; viscosity-imparting agents such as montmorillonite and pullulan; inorganic powders such as silicic acid, silicic acid anhydride, magnesium silicate, talc, kaolin, mica, bentonite, mica coated with titanium, bismuth oxychloride, zirconium oxide, magnesium oxide, zinc oxide, titanium dioxide, calcium carbonate, magnesium carbonate, iron oxide, ultramarine, Prussian blue, chromium oxide, chromium hydroxide, calamine, zeolite, and carbon black; polyamide; polyester; polyethylene; polypropylene; polystyrene; polyurethane; vinyl resin; urea resin; phenolic resin; fluororesin; silicone resin; acrylic resin; melamine resin; epoxy resin; polycarbonate resin; divinylbenzene styrene copolymer; copolymers composed of at least two types of the above monomers; polysaccharides such as celluloid, acetyl cellulose, cellulose, starch, chitin, and chitosan; proteins including silk; organic powders of scleroprotein; natural pigments such as anthraquinone, anthocyanin, chalcone, and carotenoid pigments including safflower, crocin, *Lithospermi Radix* extract, and cochineal; photosensitizing dyes of Kankoso 101, Kankoso 201, Kankoso 301, and Kankoso 401; powders of organic pigments such as Red Nos. 201, 202, 204, 205, 220, 226, 228 and 405, Orange Nos. 203 and 204, Yellow Nos. 204 and 401, and Blue No. 404; powders of organic pigments of zirconium, ballium and aluminum lakes of Red Nos. 3, 104, 106, 227, 230, 401 and 505, Orange No. 4, Yellow Nos. 4, 5, 202 and 203, Green No. 3, and Blue No. 1; and powders thereof to which are supported colors such as natural and organic synthetic pigments of *Lithospermi Radix* and safflower, as well as functional ingredients such as glycosyl rutin, glycosyl hesperidin, glycosyl arginine, esculetin, esculin, and glycosyl esculin. The amount of the above-identified substances to be incorporated into external dermal agents should not specifically be restricted as long as it does not inhibit the property and/or the activity and effect of the desired external dermal agents. Depending on purposes, the amount can be appropriately determined and it is usually in the range of 0.0001 to 99%, preferably, 0.001 to 70%, and more preferably, 0.01 to 20% to the total amount of each of the external dermal agents.

The external dermal agents incorporated with the humectant of the present invention are explained with reference to the following Experiments:

### Experiment 1: (1) Humectancy of α-glycosyl glycyrrhizin

Experiment for confirming the influence of α-glyoosyl glycyrrhizin on humectancy was conducted as follows:

### <Preparation of α-glycosyl glycyrrhizin solution>

Fifty grams of α-glycyrrhizin and 200 g of dextrin with a dextrose equivalent (DE) of 8 were dissolved by heating in 500 g of water, and the resulting solution was adjusted to give a pH of 7.0 with 2N aqueous sodium hydroxide solution, followed by enzymatic reaction at 68°C for 48 hours after the addition of 45 units/g dextrin of cyclomaltodextrin glucanotransferase derived from *Bacillus stearothermophilus,* Hayashibara Biochemical Laboratories, Inc., Okayama, Japan. After completion of the enzymatic reaction, the remaining enzyme was inactivated by heating, and the culture was filtered and purified to prepare an aqueous solution of α-glycosyl glycyrrhizin with a solid content of 10% (w/w) and a total glycyrrhizin (glycosylation degree of 64%) content of 20%, on a dry solid basis (d.s.b.). The aqueous solution was fed to a column packed with 1.5 L of "AMBERLITE XAD-7", a product name of a macroporous synthetic adsorption resin commercialized by Rohm & Hass Company, Philadelphia, PA., USA, which had been activated with an aqueous ethanol solution having a relatively high concentration, followed by washing the column with two-fold volumes of water to the column volume, eluting the ingredients adsorbed on the resin with a linear gradient of an aqueous ethanol solution with a concentration ranging from 10 to 50% (v/v), and collecting fractions rich in α-glycosyl glycyrrhizin. The fractions were pooled and in usual manner concentrated in vacuo to remove ethanol, resulting in obtaining an aqueous high α-glycosyl glycyrrhizin content solution with a solid content of about 25% (w/w), containing at least 95%, d.s.b., of α-glyoosyl glycyrrhizin but no glycyrrhizin.

### <Preparation of test sample>

An aqueous high α-glycosyl glycyrrhizin content solution, prepared by the above method, was diluted with distilled water to obtain a test sample with an α-glycosyl glycyrrhizin concentration of 0%, 0.0001%, 0.001%, 0.01%, 0.1%, 0.5%, 1%, 5%, 10% or 20%.

### <Test method>

To confirm whether these test samples have humectancy, each of which was applied on the dorsum of manus of 21 panels to evaluate the humectancy of the test samples with an index of "moisturizing feeling". The samples were evaluated based on the five grade scores; "5: excellent", "4: satisfactory", "3: passable", "2: slightly inferior", and "1: inferior". For each sample, mean value was calculated with the scores of 21 panels. The evaluation result on the feeling of use for each test sample is in Table 1, indexed with judgement symbols of "⊚", "○", "△", and "x" for scores of 5 to 4.5, 4.4 to 3.5, 3.4 to 2.5, and not higher than 2.4, respectively.

**Table 1**

| Concentration of α-glycosyl glycyrrhizin | Evaluation of the moisturizing feeling |
|---|---|
| 0 | x |
| 0.0001 | x |
| 0.001 | Δ |
| 0.01 | ○ |
| 0.1 | ○ |
| 0.5 | ⊚ |
| 1 | ⊚ |
| 5 | ⊚ |
| 10 | ⊚ |
| 20 | △ |

As evident from the result in Table 1, the test samples containing α-glycosyl glycyrrhizin, preferably, containing at least 0.001% of α-glycosyl glycyrrhizin were evaluated as possessing a desired moisturizing feeling. The higher the content of α-glycosyl glycyrrhizin the more the moisturizing feeling becomes remarkable, and thus the compositions containing 0.001% or higher but 20% or lower of α-glycosyl glycyrrhizin are judged to have a moisture-retaining activity. In the case of reaching to a concentration of 20% α-glycosyl glycyrrhizin, some panels evaluated that such a test sample becomes sticky and lowers in evaluation of moisturizing feeling, compared with other test samples with a concentration of 1 to 10% of α-glycosyl glycyrrhizin, resulting in different judgements among the panels. Experiment 2: (2) Humectancy of α-glycosyl glycyrrhizin

A test for confirming the influence of different percentages of glycyrrhizin and α-glycosyl glycyrrhizin on humectancy.

### <Preparation of test sample>

The aqueous high α-glycosyl glycyrrhizin content solution, prepared in Experiment 1, and glycyrrhizin were mixed into an aqueous solution with a percentage of α-glycosyl glycyrrhizin to give a total α-glycosyl glycyrrhizin and glycyrrhizin (called "total glycyrrhizin", hereinafter) percentage of 40%, 50%, 60%, 70%, 80%, or 90%, d.s.b., and diluted to prepare test samples with a total glycyrrhizin concentration of 0.01%. These test samples were subjected to a panel test with 20 panels similarly as the method in Experiment 1 and evaluated whether the samples have a desired humectancy. The result is in Table 2.

**Table 2**

| Percentage (%) of α-glycosyl glycyrrhizin to the total glycyrrhizin | Evaluation of moisturizing feeling |
|---|---|
| 40 | x |
| 50 | Δ |
| 60 | ○ |
| 70 | ○ |
| 80 | ○ |
| 90 | ○ |

As evident from the result in Table 2, the test samples with percentages of at least 50%, preferably, at least 60% of α-glycosyl glycyrrhizin in the total glycyrrhizin were evaluated to have an ability of imparting a satisfactory moisturizing feeling. The higher the percentage the more the moisturizing feeling becomes remarkable, and thus the compositions with a percentage of at least 50% of α-glyoosyl glycyrrhizin in the total glycyrrhizin are judged to have a desired moisture-retaining ability.

### Experiment 3: Powdery foundation

A powdery foundation containing α-glycosyl glycyrrhizin was prepared and evaluated for close adhesiveness, makeup finish feeling, humectancy, and makeup retainability. The powdery foundation was prepared based on the formulation in Table 3 in such a manner of mixing Ingredients A with a Henshel mixer, and admixing with Ingredients B previously dissolved to homogeneity by heating, followed by mixing and pulverizing the resulting mixture and compressing and forming the mixture in a middle plate.

**Table 3**

| Ingredients | Composition (part by weight) | |
|---|---|---|
| | The present invention | Reference for example |
| Silicon-treated titanium dioxide | 8.80 | 8.80 |
| Silicon-treated talc | 15.29 | 15.29 |
| Silicon-treated mica | 8.80 | 8.80 |
| LIPIDURE^{®}-SERICIPE^{*1} | 37.80 | 39.60 |
| Poly(methyl methacrylate) | 8.80 | 8.80 |
| Titanium dioxide particle | 4.40 | 4.40 |
| Powdery humectant containing the α-glycosyl glycyrrhizin obtained by the later described method in Example 1 | 1.80 | - |
| Silicon-treated yellow iron oxide | 1.76 | 1.76 |
| Silicon-treated red iron oxide | 0.45 | 0.35 |
| Silicon-treated black iron oxide | 0.10 | 0.09 |
| Dimethicone | 4.49 | 4.49 |
| Trimethylsiloxysilicate | 1.50 | 1.50 |
| ELDUW^{®}-PS-304^{*2} | 1.00 | 1.00 |
| 2,2-Dimethyl propanediol di(2-ethylhexanoate | 1.00 | 1.00 |
| Squalane | 4.00 | 4.00 |
| Tocopherol | 0.01 | 0.01 |
| Preservative (Methylparaben, propylparaben) | q.s. | q.s. |
| Total | 100.00 | 100.00 |

| | | |
|---|---|---|
| *1: NOF Corporation, Tokyo, Japan *2: Ajinomoto Co., Inc., Tokyo, Japan | | |

Using the powder foundations of the present invention and the reference for example formulated with the composition in Table 3, they were evaluated on their feeling of use by 20 panels. The evaluation was carried out by applying the foundation of the present invention to the right side of each panel's face and applying the one of the reference for example to the left side of each panel's face, and studying on the four items of "close adhesiveness to the skin", "feeling of makeup finish", "moisturizing feeling", and "durability of makeup" 5-hours after makeup, in total. Comparing the foundation of the present invention with the one of the reference for example in terms of the above respective items, it was organoleptically evaluated based on three grade scores; "3: the foundation of the present invention is superior to the one of the reference for example", "2: even", and "1: the foundation of the reference for example is superior to the one of the present invention". The mean values ranging from 2.9 to 3.0, 2.5 to 2.8, 2.4 to 1.6, and not higher than 1.5 are respectively expressed with symbols for judgement of "⊚", "○", "△" and "x", and the evaluation results on the feeling of use for respective items are in Table 4.

**Table 4**

| Evaluation items for the feeling of use | The present invention |
|---|---|
| Close adhesiveness | ⊚ |
| Feeling of makeup finish | ○ |
| Moisturizing feeling | ⊚ |
| Durability after makeup | ○ |

As evident from Table 4, comparing with the foundation formulated with no α-glycosyl glycyrrhizin as the reference for example, the one of the present invention containing α-glycosyl glycyrrhizin in an amount of 1.8% to the total amount of the cosmetic was evaluated to be superior in close adhesiveness to the skin, feeling of makeup finish, moisturizing feeling, and durability after makeup.

### Experiment 4: Sunscreen cream

A sunscreen cream containing α-glucosyl glycyrrhizin was prepared and evaluated for close adhesiveness to the skin, feeling of makeup finish, moisturizing feeling, and durability after makeup. Based on the composition as shown in Table 5, the sunscreen cream was prepared by weighing Ingredients A in a container suitable for enclosing all the ingredients, dissolving it by heating, adding to the resulting solution Ingredients B previously pulverized to homogeneity, mixing the mixture to homogeneity, further adding to the resulting mixture Ingredients C previously dissolved to homogeneity by heating in another container, allowing the mixture thus obtained to homogeneity with a homogenizer, and cooling it to ambient temperature while stirring.

**Table 5**

| | Ingredients | Composition (%) | |
|---|---|---|---|
| | | The present invention | Reference for example |
| | Sorbitan sesquioleate | 2.00 | 2.00 |
| | Dimethicone copolyol | 1.50 | 1.50 |
| | Isotridecyl isononanoate | 4.00 | 4.00 |
| A | Microcrystalline wax | 6.00 | 6.00 |
| | Propylparaben | 0.10 | 0.10 |
| | Phenyltrimethicone | 7.00 | 7.00 |
| | Octanium-18 bentonite | 1.00 | 1.00 |
| | Decamethyl cyclopenta siloxane | 15.00 | 15.00 |
| | Titanium dioxide particle | 5.00 | 5.00 |
| | Zinc oxide particle | 10.00 | 10.00 |
| B | Mica | 4.00 | 4.00 |
| | Talc | 5.72 | 5.72 |
| | Red iron oxide | 0.20 | 0.20 |
| | Refined water | 32.28 | 32.28 |
| | 1,3-Butyleneglycol | 2.00 | 5.00 |
| C | Powdery humectant prepared by the later described method in Example 3 | 3.00 | - |
| | Sodium chloride | 1.00 | 1.00 |
| | Methylparaben | 0.20 | 0.20 |
| | Total | 100.00 | 100.00 |

Using either of the sunscreen creams of the present invention and the reference for example in Table 5, 20 panels evaluated the levels of "extensibility", "stickiness", and "moisturizing feeling" when applied in such a manner of their usual use of sunscreen creams, based on five grade scores; "5: superior", "4: satisfactory", "3: passable", "2: slightly inferior", and "1: inferior", followed by calculating the mean values of 20 panels for each items. The mean values ranging from 5 to 4.5, 4.4 to 3.5, 3.4 to 2.5, and not higher than 2.4 are respectively expressed with symbols for judgement of "⊚", "○", "Δ" and "x", and the evaluation results on the feeling of use for respective items are in Table 6.

**Table 6**

| Evaluation items of the feeling of use | The present invention | Reference for example |
|---|---|---|
| Extensibility | ○ | ○ |
| Stickiness | ○ | △ |
| Moisturizing feeling | ⊚ | △ |

As evident from Table 6, the sunscreen cream formulated with no α-glucosyl glycyrrhizin of the reference for example was evaluated to be slightly inferior in stickiness and moisturizing feeling, while the one of the present invention containing 3% of α-glucosyl glycyrrhizin was evaluated to have a property of imparting a satisfactory moisturizing feeling even though it does not impart stickiness. The result revealed that the sunscreen cream formulated according to the present invention has a superior skin-beautifying effect.

### Experiment 5: (1) Influence of α-glucosyl glycyrrhizin on melanin pigmentation

Using a sunscreen cream prepared according to the formulation in Table 5, the influence of α-glucosyl glycyrrhizin on pigmentation induced by ultraviolet rays was confirmed. Twenty panels were applied on the skin surfaces inside of the upper arms of their right hands with a sunscreen cream previously prepared based on the composition ratio of the reference for example in Table 5, and then irradiated with ultraviolet rays to determine the irradiation dose of ultraviolet rays sufficient to induce erythema within 24 hours after irradiation, and irradiated on the skin surfaces (two parts, 1 cm x 1 cm each) inside of the upper arms of their left hands with ultraviolet rays at the dose determined in the above once a day for three days. For 30 days from the day 3 before initiating the irradiation of ultraviolet rays, one of the above two parts irradiated with ultraviolet rays was applied with the sunscreen cream formulated according to the present invention and the other part was applied with the one formulated according to the reference for example at a dose of three times a day, respectively. The parts irradiated with ultraviolet rays were macroscopically observed over time to confirm the degree of pigmentation, revealing that, for 16 panels out of 20 panels, the level of pigmentation in the part applied with the sunscreen cream of the present invention was lower than that applied with the one of the reference for example. No difference was observed between the sunscreen creams of the present invention and the reference for example in the remaining 4 panels. Scaling of the death skin caused by the irradiation of ultraviolet rays was observed in the parts irradiated with ultraviolet rays, however, the level of which applied with the sunscreen cream of the present invention was lower than that applied with the one of the reference for example, and the erythema in the parts irradiated with ultraviolet rays and applied with the sunscreen cream of the present invention was disappeared within a shorter period of time than that applied with the one of the reference for example. Because of this, it was judged that α-glucosyl glycyrrhizin has a skin-whitening effect of inhibiting the skin pigmentation and also has an action of activating cells to promote the recovery of normal skin from conditions damaged by ultraviolet rays.

### Experiment 6: (2) Influence of α-glycyrrhizin on melanin pigmentation

Fifty grams of the powdery humectant containing α-glucosyl glycyrrhizin prepared in the later described Example 2 was redissolved in 500 g of refined water, and fed to a column packed with 1.5 L of "AMBERLITE XAD-7", a product name of a macroporous synthetic adsorption resin commercialized by Rohm & Hass Company, Philadelphia, PA., USA. The column was washed with two-fold volumes of water to the column volume and fed with a linear gradient of an aqueous ethanol solution with an ethanol concentration ranging from 10 to 50% (v/v) to elute the components adsorbed on the resin, followed by collecting fractions containing glycyrrhizin and α-glycosyl glycyrrhizin. The fractions were pooled and in usual manner concentrated in vacuo to remove ethanol, and dried by spraying to prepare an α-glycosyl glycyrrhizin powder containing, on a dry solid basis, 60% of α-glucosyl glycyrrhizin and 40% of other α-glycosyl glycyrrhizin. The powder was mixed with the powdery humectant rich in α-glucosyl glycyrrhizin in Example 3 to give a percentage of α-glucosyl glycyrrhizin against α-glycosyl glycyrrhizin to be 60%, 70%, 80%, or 90% for use as test samples. Except for using 0.5 part by weight of any one of the test samples in place of the α-glycosyl glycyrrhizin used in the composition of the sunscreen cream of the present invention in Table 5, sunscreen creams were prepared with the same ingredients and the same composition ratios as in the table. These sunscreen creams were subjected to a panel test with 21 panels by the same test and evaluation method as used in Experiment 5, except for applying five parts, 1 cm x 1 cm each, inside of the upper arms of their left hands with the test creams. As a reference for example, the cream with the composition as shown in the reference for example in Table 5 was used.

As a result, all the skin parts of 17 panels out of 21 panels, applied with the four types of sunscreen creams incorporated with the α-glycosyl glycyrrhizin of the present invention used in this experiment, were low in pigmentation compared with those applied with the one of the reference for example. The higher the percentage of α-glucosyl glycyrrhizin to α-glycosyl glycyrrhizin the more the pigmentation tends to decrease. The remaining four panels gave no difference in pigmentation when applied with any of the creams of the present invention and the reference for example. Although scaling of the death skin caused by the irradiation of ultraviolet rays was observed in the parts received with the irradiation, the levels of which applied with the four types of sunscreen creams of the present invention were lower than that applied with the one of the reference for example. The amount of scaling decreased as the increase of the percentages of 60%, 70%, and 80%, particularly, in the case of 90%, it was distinctly low as compared with others, and the effect of disappearance of erythema in the skin parts, irradiated with ultraviolet rays, had the same tendency as in the others.

Based on the results in Experiments 1 to 6, it was revealed that α-glycosyl glycyrrhizin has an advantageous moisturizing activity and an activity of inhibiting the pigmentation induced by ultraviolet rays and of preventing inflammation, and it can be advantageously used in external dermal agents as a humectant, skin-whitening agent, anti-inflammatory, skin-protecting agent, retrogradation-preventing agent, improver for crease or fine crease, and agent for inhibiting the generation of crease or fine crease.

The present invention is explained with reference to the following examples in more detail but it should not be limited thereto:

### Example 1: Humectant

Fifty grams of glycyrrhizin and 200 g of dextrin with a DE of 8 were dissolved by heating in 500 g of water, and the solution was adjusted to pH 7.0 with 2N-sodium hydroxide and admixed with 45 units/g dextrin of cyclomaltodextrin glucanotransferase derived from *Bacillus stearothermophilus,* commercialized by Hayashibara Biochemical Laboratories, Inc., Okayama, Japan, followed by enzymatic reaction at 68°C for 48 hours. After completion of enzymatic reaction, the reaction mixture was heated to inactivate the remaining enzyme and filtered. The filtrate was fed to a column packed with 1.5 L of "AMBERLITE XAD-7", a product name of a macroporous synthetic adsorption resin commercialized by Rohm & Hass Company, Philadelphia, PA., USA, which had been activated by an aqueous ethanol solution with a relatively high concentration, followed by washing the column with two-fold volumes of water to the column volume and eluting the ingredients adsorbed on the resin with three liters of a 50% (v/v) aqueous ethanol solution. In usual manner, the eluate was concentrated in vacuo to remove ethanol and dried to obtain 78 g of a powdery humectant rich in α-glycosyl glycyrrhizin. The powdery humectant contained about 85% of α-glycosyl glycyrrhizin and glycyrrhizin in total, where the content of α-glycosyl glycyrrhizin was 64% to the total amount of glycyrrhizin. The product can be advantageously used as a skin-whitening agent, as well as a humectant for external dermal agents.

### Example 2: Humectant

Fifty grams of a powdery α-glycosyl glycyrrhizin prepared in accordance with the method in Example 1 was redissolved in 500 g of refined water, adjusted to pH 4.5, and enzymatically reacted after the addition of 0.1 g of "GLUCOZYME", a product name of a glucoamylase specimen commercialized by Amano Enzyme, Inc., Tokyo, Japan. After completion of the enzymatic reaction, the reaction mixture was heated to inactivate the remaining enzyme and filtered. Similarly as in Example 1, the filtrate was fed to a column packed with 1.5 L of "AMBERLITE XAD-7", a product name of a macroporous synthetic adsorption resin commercialized by Rohm & Hass Company, Philadelphia, PA., USA, which had been activated by an aqueous ethanol solution with a relatively high concentration, followed by washing the column with two-fold volumes of water to the column volume and eluting the ingredients adsorbed on the resin with three liters of a 50% (v/v) aqueous ethanol solution. In usual manner, the eluate was concentrated in vacuo to remove ethanol and dried to obtain 24 g of a powdery humectant rich in α-glucosyl glycyrrhizin. The powdery humectant had a composition of, on a dry solid basis, about 58% of α-glucosyl glycyrrhizin, 39% of glycyrrhizin other than the α-glucosyl glycyrrhizin, and 3% of glycyrrhizin, and other ingredients were not almost detected. The product can be advantageously used as a skin-whitening agent, as well as a humectant for external dermal agents.

After dissolving in water, the product was orally administered to mice for acute toxicity test. As a result, it induced neither mouse death nor any abnormal conditions, and the LD₅₀ was estimated to be 1 g/kg body weight or higher.

### Example 3: Humectant

Fifty grams of a powder containing α-glucosyl glycyrrhizin prepared in accordance with the method in Example 2 was redissolved in 500 g of refined water, and similarly as in Example 1, the solution was fed to a column packed with 1.5 L of "DIAION HP-20", a product name of a macroporous synthetic adsorption resin commercialized by Mitsubishi Chemical Corporation, Tokyo, Japan, which had been activated by an aqueous ethanol solution with a relatively high concentration, followed by washing the column with two-fold volumes of water to the column volume and eluting the ingredients adsorbed on the resin with a linear gradient of an aqueous ethanol solution with a concentration ranging from 10 to 50% (v/v) to elute the ingredients adsorbed on the resin, followed by collecting fractions containing α-glucosyl glycyrrhizin. The fractions were pooled and in usual manner concentrated *in vacuo* to remove ethanol, and granulated by spraying to obtain 19 g of a powdery humectant rich in α-glucosyl glycyrrhizin. The product contained about 92%, d.s.b., of α-glucosyl glycyrrhizin and glycosyl glycyrrhizin other than the α-gluoosyl glycyrrhizin but it contained almost no other ingredients. The product can be advantageously used as a skin-whitening agent, as well as a humectant for external dermal agents.

### Example 4: Washing foam

Based on the formulation below, the total ingredients were dissolved by heating at 75 to 85°C and the solution was gradually cooled to ambient temperature while stirring to obtain the captioned product. Since the product contains α-glycosyl glycyrrhizin, it is a washing foam capable of forming a creamy foam and having both a refreshing washing-feeling just after use and a durable moisturizing-feeling. The blending quantities of the material ingredients in the following Examples are all expressed by part by weight.

| | |
|---|---|
| N-Acyl-L-sodium glutamate | 20 |
| N-Coconut oil fatty acid/ sodium hydrogenated tallow-L-glutamate | 2 |
| Palm kernel oil fatty acid diethanolamide | 1 |
| Polyoxyethylene sorbitan monococoate (20 E.O.) | 5 |
| 1,3-Butyleneglycol | 4 |
| Propyleneglycol | 27 |
| Polyethyleneglycol monostearate | 1 |
| Humectant prepared by the method in Example 2 | 2 |
| p-Hydroxybenzoate ester (paraben) | 0.3 |

To the above ingredients, refined water was added to give a total amount of 100.

### Example 5: Rouge

Based on the formulation below, to a mixture, which had been previously prepared by mixing and dissolving Ingredients A by stirring at 85°C, was added another mixture obtained by adding Ingredients B to a part of caster oil and treating the resultant with a roller, followed by dispersing the resulting mixture to homogeneity. The mixture thus obtained was poured into a mold and promptly cooled to form a stick-shaped product as the captioned product. Since the product contains α-glycosyl glycyrrhizin, it is a rouge having a smooth extensibility, satisfactory makeup finish, and humectancy, and retains the lip in a moistened condition.

### <Ingredients A>

| | |
|---|---|
| Castor oil | 25 |
| Cetyl 2-ethylhexanoate | 15 |
| Lanolin | 11 |
| Isopropyl myristate | 10 |
| Candelilla wax | 9 |
| Paraffin | 8 |
| Beeswax | 5 |
| Carnauba wax | 5 |
| Propyl parahydroxybenzoate | q.s. |

### <Ingredients B>

| | |
|---|---|
| Humectant prepared by the method in Example 3 | 4 |
| Titanium dioxide | 6 |
| Red No. 202 | 0.6 |
| Red No. 201 | 0.2 |
| Red No. 223 | 0.2 |

### Example 6: Eye shadow

Based on the formulation below, Ingredients A was mixed with a Henshel mixer and admixed with Ingredients B which had been dissolved to homogeneity by heating. The mixture was mixed for pulverization and subjected to compression molding in a middle plate to obtain an eye shadow. Since the product contains α-glycosyl glycyrrhizin, it has a satisfactory close adhesiveness and feeling of makeup finish, imparts a satisfactory moisturizing feeling in eyelids and tails of eyes when applied thereto, and has a satisfactory feeling of use.

### <Ingredients A>

| | |
|---|---|
| Silicon-treated talc | 27 |
| Silicon-treated titanium dioxide | 9 |
| Boron nitrite | 9 |
| Silicon-treated (talc/potassium silicofluoride) burned product | 9 |
| | |
| "SICOPAL PINK", commercialized by BASF Japan, Ltd., Tokyo, Japan | 9 |
| | |
| Humectant prepared by the method in Example 1 | 9 |
| | |
| Methylparaben | 1 |

### <Ingredients B>

| | |
|---|---|
| Dimethicone | 5 |
| Neopentylglycol dioctanoate | 1 |
| Squalane from plant | 4 |
| Tocopherol | 0.01 |
| Propylparaben | 0.01 |

### Example 7: Makeup base

Based on the formulation below, Ingredients C was added to and dispersed in Ingredients B which had been previously dissolved by heating, and the mixture was mixed to homogeneity after admixed and emulsified with Ingredients A which had been previously dissolved by heating in another container. The resulting mixture was cooled to ambient temperature while stirring to obtain the captioned product. Since the product contains α-glycosyl glycyrrhizin, it is a makeup base which has an improved close adhesive compatibility with foundations, substantially does not cause makeup deterioration, and has a durable moisturizing feeling and preferable feeling of use.

### <Ingredients A>

| | |
|---|---|
| Refined water | 49 |
| 1,3-Butyleneglycol | 2 |
| Glycerin | 1 |
| | |
| Humectant prepared by the method in Example 3 | 1 |
| | |
| Montmorillonite | 0.5 |
| "PULLULAN PI-20", a product name of pullulan commercialized by Hayashibara Shoji Inc., Okayama, Japan | 0.1 |
| | |
| Sodium hexametaphosphate | 0.05 |
| Disodium ethylenediaminetetraacetate | 0.05 |
| Sodium hydroxide | 0.1 |

### <Ingredients B>

| | |
|---|---|
| Stearic acid | 1 |
| Palmitic acid | 1 |
| Isostearyl palmitate | 3 |
| Petrolatum | 1 |
| Dimethyl polysiloxane (6CS) | 10 |
| Liquid paraffin | 10 |
| POE Glyceryl monostearate | 1 |
| Glyceryl monostearate | 1 |
| Antioxidant | 0.05 |
| Preservative | 0.2 |
| Flavor | 0.15 |

### <Ingredients C>

| | |
|---|---|
| Titanium dioxide | 4 |
| Sericite | 8 |
| Yellow oxide of iron | 0.1 |
| Titanium dioxide particle | 5 |
| Cobalt titanium oxide | 0.7 |

### Example 8: Milky lotion

Based on the formulation below, a milky lotion was prepared in usual manner. Since the product contains α-glycosyl glycyrrhizin, it is a milky lotion having an improved humectancy, comfortable feeling when applied, non-stickiness after application, and satisfactory feeling of use. The product can be advantageously used for preventing stimulation and itching in the skin, as well as for skin-whitening and treating/preventing pigmentation such as chloasma, freckle, and sunburn, and retrogradation of the skin.

| | |
|---|---|
| Stearic acid | 2.5 |
| Cetanol | 1.5 |
| Petrolatum | 5 |
| Liquid paraffin | 10 |
| Polyoxyethylene (10 mole) oleate | 2 |
| Propylparaben | 0.1 |
| *d1*-α-Tocopheryl acetate | 0.5 |
| Flavor | 0.2 |
| Polyethyleneglycol (1500) | 3 |
| Triethanolamine | 1 |
| Humectant prepared by the method in Example 3 | 0.5 |
| "AA2G", a product name of L-ascorbic acid 2-glucoside, commercialized by Hayashibara Biochemical Laboratories, Inc., Okayama, Japan | 2 |
| "TORNARE", a product name of a saccharide composition containing saccharide derivatives of α,α-trehalose, commercialized by Hayashibara Biochemical Laboratories, Inc., Okayama, Japan | 1.5 |

| | |
|---|---|
| Deionized water was added to the above ingredients in an adequate amount sufficient to volume up to 100. | |

### Example 9: Lotion

Based on the formulation below, a lotion was prepared in usual manner. Since the product contains α-glycosyl glycyrrhizin, it is a lotion having an improved humectancy and feeling of use without causing unsatisfactory stickiness when applied to the skin. The product can be advantageously used for preventing/treating skin roughness, stimulation and itching in the skin, pigmentation such as chloasma, freckle, sunburn, and retrogradation of the skin. Since the product has an improved humectancy and a lesser stimulation to the skin, it can be used without fear of inducing hypersensitivity.

| | |
|---|---|
| Humectant prepared by the method in Example 2 | 0.5 |
| Citric acid | 0.1 |
| Sodium citrate | 0.3 |
| Glycerol | 2.0 |
| Ethanol | 5.0 |
| Kankoso 201 | 0.0001 |
| Ethylparaben | 0.1 |

| | |
|---|---|
| Refined water was added to the above ingredients in an adequate amount sufficient to volume up to 100. | |

### Example 10: Cosmetic cream

Based on the formulation below, Ingredients A was dissolved by heating in usual manner and added to Ingredients B, and the mixture was emulsified by a homogenizer and mixed by stirring into a cosmetic cream. Since the product contains α-glycosyl glycyrrhizin, it is a cosmetic cream with a satisfactory feeling of use without causing unsatisfactory stickiness when applied to the skin. The product can be advantageously used for preventing stimulation and itching in the skin, and treating/preventing pigmentation such as chloasma, freckle, and sunburn.

### <Ingredients A>

| | |
|---|---|
| Polyoxyethylene glyceryl monostearate | 2 |
| Glyceryl monostearate, self-emulsifying | 5 |
| Behenyl alcohol | 1 |
| Eicosatetraenoic acid | 2 |
| Liquid paraffin | 5 |
| Glyceryl trioctanoate | 10 |
| Preservative | q.s. |

### <Ingredients B>

| | |
|---|---|
| Humectant prepared by the method in Example 2 | 2 |
| *dl*-sodium lactate | 5 |
| 1,3-Butylene glycol | 5 |
| Carrot extract | 1 |
| Refined water | 65 |
| Flavor | q.s. |
| Total | 100 |

### Example 11: Hair tonic

Based on the formulation below, a hair tonic was prepared in usual manner. Since the product contains α-glycosyl glycyrrhizin, it is a hair tonic with a satisfactory feeling of use without causing unsatisfactory stickiness to the scalp. The product has a satisfactory hair setting ability and you can easily comb your hair after applying.

| | |
|---|---|
| Ethanol | 50 |
| Polyoxyethylene (8 mole) oleate | 1.5 |
| Isostearyl pivalate (*Hinokitiol*) | 0.1 |
| Humectant prepared by the method in Example 3 | 1.0 |
| Kankoso 301 | 0.01 |
| "TORNARE", a product name of a saccharide composition containing saccharide derivatives of α,α-trehalose, commercialized by Hayashibara Biochemical Laboratories, Inc.,Okayama, Japan | 7 |
| α-Glycosyl glycyrrhizin powder prepared by the method in Example 1 | 3 |
| Ethylparaben | 0.1 |
| Flavor | 0.05 |

| | |
|---|---|
| Deionized water was added to the above ingredients in an adequate amount sufficient to volume up to 100. | |

### Example 12: Dentifrice

Based on the formulation below, Ingredients A was mixed to homogeneity by heating and successively admixed with Ingredients B and C, and the resulting mixture was mixed to homogeneity and cooled while stirring to obtain the captioned product. Since the product contains α-glycosyl glycyrrhizin, it is a dentifrice which has a satisfactory formability, imparts a fresh feeling after washing your teeth, and removes plaque well.

### <Ingredients A>

| | |
|---|---|
| *dl*-*α*-Tocopheryl acetate | 0.1 |
| Humectant prepared by the method in Example 3 | 0.5 |
| Refined water | 26.5 |
| Glycerin | 10 |
| "TORNARE", a product name of a saccharide composition containing saccharide derivatives of α,α-trehalose, commercialized by Hayashibara Biochemical Laboratories, Inc., Okayama, Japan | 30 |
| Carrageenan | 0.5 |
| Sodium carboxymethylcellulose | 0.3 |

### <Ingredients B>

| | |
|---|---|
| Dicalcium phosphate | 20 |
| Hydroxyapatite | 5 |
| Calcium carbonate | 5 |

### <Ingredients C>

| | |
|---|---|
| Sodium lauryl sulfate | 1.5 |

### Example 13: Bath salt

Based on the formulation below, all the ingredients were mixed to homogeneity and tabletted into a bath salt. Since the product contains α-glycosyl glycyrrhizin, it is a bath salt having a satisfactory feeling of use without causing roughness and stickiness in the skin after taking bath.

| | |
|---|---|
| Sodium sulfate | 44 |
| Sodium bicarbonate | 14 |
| Sodium carbonate | 7 |
| Succinic acid | 21 |
| Humectant prepared by the method in Example 1 | 5 |
| "TORNARE", a product name of a saccharide composition containing saccharide derivatives of α,α-trehalose, commercialized by Hayashibara Biochemical Laboratories, Inc.,Okayama, Japan | 3 |
| Lubricant | q.s. |
| Pigment | q.s. |
| Flavor | q.s. |
| Total | 100 |

### Example 14: Toilet soap

Based on the formulation below, a toilet soap was prepared. The product is a soap having both an improved moisturizing effect by L-ascorbic acid 2-glucoside and α-glycosyl glycyrrhizin and a satisfactory feeling of use without causing roughness in the skin after use. Since the oxidation and the decomposition of the soap base, emulsifying substance, flavor, pigment, etc., are well inhibited and the occurrence of browning, discoloration, and off-flavor are prevented for a relatively long period of time, the quality of the product can be stably retained for a relatively long period of time.

| | |
|---|---|
| Neat soap obtained by subjecting beef tallow and coconut oil in a ratio of 4:1 by weight to conventional saponification and salting out | 95.5 |
| Humectant prepared by the method in Example 1 | 0.5 |
| "TREHALOSE FOR COSMETICS", a product name of hydrous α,α-trehalose, commercialized by Hayashibara Biochemical Laboratories, Inc., Okayama, Japan | 1 |
| "AA2G", a product name of L-ascorbic acid 2-glucoside, commercialized by Hayashibara Biochemical Laboratories, Inc., Okayama, Japan | 0.5 |
| White sugar | 0.5 |
| "αG RUTIN", a product name of a glycosyl rutin, commercialized by Toyo Sugar Refining Co., Ltd., Japan | 0.5 |
| Maltitol | 1 |
| Kankoso 201 | 0.001 |
| Flavor | q.s. |
| Total | 100 |

### Possibility of Industrial Applicability

The α-glycosyl glycyrrhizin used in the present invention has a satisfactory moisturizing activity and it is used to prepare external dermal agents having a satisfactory feeling of use and preferable makeup finish, imparts a desired softness and tension to the skin, and improves humectancy in the skin without causing unfavorable stickiness. Since the α-glycosyl glycyrrhizin used in the present invention has also an improved skin-whitening activity, cell-activating activity, and skin-retrogradation-preventing activity, it has advantageous features of skin-whitening activity, skin-protecting/strengthening activity, and skin-retrogradation-preventing activity.

## Claims

1. A humectant comprising α-glycosyl glycyrrhizin as an effective ingredient.

2. The humectant of claim 1, wherein said α-glycosyl glycyrrhizin is one or more members selected from the group consisting of α-glycosyl glycyrrhizin composed of glycyrrhizin to which at least an equimolar glucose residue is bound via the α-linkage.

3. The humectant of claim 1 or 2, which contains α-glycosyl glycyrrhizin and glycyrrhizin in a total amount of at least 80% by weight, on a dry solid basis.

4. The humectant of any one of claims 1 to 3, which contains α-glycosyl glycyrrhizin in an amount of at least 60% by weight of the total amount of α-glycosyl glycyrrhizin and glycyrrhizin.

5. The humectant of any one of claims 1 to 4, wherein at least 60% by weight of said α-glycosyl glycyrrhizin is in the form of α-glucosyl glycyrrhizin.

6. The humectant of any one of claims 1 to 5, which is used as a skin-whitening agent.

7. An external dermal agent, comprising any one of the humectants of claims 1 to 6.

8. The external dermal agent of claim 7, which contains any one of the humectants in an amount of 0.001 to 20% by weight of the total amount of said agent.

9. The external dermal agent of claim 7 or 8, which is a member selected from the group consisting of basic cosmetics, makeup cosmetics, body cosmetics, hair cosmetics, sunburn/suntan-preventing cosmetics, soaps, bath salts, cosmetics for oral health, quasi-drugs, and pharmaceuticals.
